# EUROPEAN PATENT APPLICATION

(11) **EP 2 260 855 A1**
(43) Date of publication of application: **15.12.2010**
(21) Application number: 09718219.0
(22) Date of filing: 05.03.2009
(51) Int. Cl.: A61K 36/18, A61P 3/00, A61P 3/06, A61P 3/10, A61P 15/08, A61P 17/16, A61P 19/02, A61P 29/00, A61P 31/04, A61P 35/00, A61P 43/00

(54) **COMPOSITION CONTAINING AQUEOUS EXTRACT OF ASHWAGANDA LEAVES AS THE ACTIVE INGREDIENT AND METHOD OF PRODUCING THE SAME**

(30) Priority: 06.03.2008 JP 2008055828
(71) Applicant: National Institute of Advanced Industrial Science and Technology, Tokyo 100-8921 (JP); Igene Therapeutics, Inc., Tokyo 103-8355 (JP)
(72) Inventor: WADHWA, Renu, Tsukuba-shi Ibaraki 305-8562 (JP); KAUL, Sunil, Tsukuba-shi Ibaraki 305-8562 (JP); WIDODO, Nashi, Tsukuba-shi Ibaraki 305-8562 (JP); TAKAGI, Yasuomi, Tokyo 103-8355 (JP)
(74) Representative: Polypatent
(86) International application number: PCT/JP2009/054161
(87) International publication number: WO 2009/110546

(57) **Abstract**

It is an object of the present invention to provide a composition comprising, as an active ingredient, a pharmacologically elective extract of Ashwagandha leaves obtained without the use of an organic solvent, and a method for producing the same. Such object is attained by providing a composition for inhibiting or preventing aging, treating or preventing tumor or cancer, promoting the proliferation of normal cells or prolonging the life of the same, inducing the proteasomal activity of normal cells, protecting normal cells from stress caused by oxidation or UV irradiation, killing cancer cells or tumor cells or inhibiting the proliferation of the same, or increasing the sensitivity of cancer cells to an anticancer agent which comprises, as an active ingredient, a water extract of Ashwagandha leaves, and a method for producing such composition comprising: a step of adding Ashwagandha leaves to water and treating the same at 20°C to 70°C for 6 to 100 hours; or a step of adding Ashwagandha leaves to water, heating the mixture to 70°C to 100°C, and then letting the resultant cool to 1°C to 30°C.

## Description

### Cross-Reference to Related Application

This patent application claims priority from Japanese Patent Application No. 2008-055828 filed on March 6, 2008, the disclosure of which is incorporated herein by reference.

### Technical Field

The present invention relates to a composition comprising, as an active ingredient, a water extract of Ashwagandha leaves. More particularly, the present invention relates to a composition comprising, as an active ingredient, a water extract of Ashwagandha leaves, for inhibiting or preventing aging, treating or preventing tumor or cancer, promoting the proliferation of normal cells or prolonging the life of the same, inducing the proteasomal activity of normal cells, protecting normal cells from stress caused by oxidation or UV irradiation, killing cancer cells or tumor cells or inhibiting the proliferation of the same, or increasing the sensitivity of cancer cells to an anticancer agent.

Further, the present invention relates to a method for producing a composition comprising, as an active ingredient, a water extract of Ashwagandha leaves comprising a step of adding Ashwagandha leaves to water and treating the same under conditions suitable for extraction of the water-soluble components contained in Ashwagandha leaves.

### Background Art

Ayurveda is a 5000-year-old system of medicine originating from India, which is one of the oldest system in the world. The term "Ayurveda" is derived from Classical Sanskrit. The word "ayu" means "life," the word "veda" means "knowledge," and this term is often translated as "the science of life." It is a method of health maintenance that has been thoroughly thought out and developed by prophets and natural scientists over many centuries. Research and experiments have been repeated, numerous discussions have taken place, and thorough consideration has given to the matter. Teachings have been passed down from masters to their apprentices over several thousand years, and very through guidance was written in Sanskrit (i.e., the classical language of India) from the fifth to the sixth centuries B.C. Ayurveda is said to be effective for prevention of diseases, promotion of rejuvenation, and life extension. In addition, scientists have recently begun to verify the effects of Ayurvedic medical herbs in clinical practice all over the world.

Ashwagandha (*Withania somnifera*) is the most prominent medical herb used in Ayurveda. It is referred to as "the queen of Ayurveda," "the bounty from Ayurveda," and the like. It is an evergreen shrub found in the a-rid zones of India, and it grows to a height of approximately 1 m. The tree keeps its leaves all year round and grows in fertile, adequately moist, and well-drained soil. The roots thereof have been used for over 3,000 years. Ashwagandha is also referred to as: *Withania somnifera* (Latin), Ashwagandha (Sanskrit), Ashgandha (Hindi), Indian Ginseng, or Winter Cherry (English).

Ashwagandha is classified as a restorative agent (the most trusted Ayurvedic medical herb), and it has been extensively evaluated as allowing health promotion, rejuvenation promotion, and life extension. Efficacies that are generally trusted are as described below.

### (Anti-stress effects)

Ashwagandha is characterized among Ayurrvedic herbs as a medical herb having anti-stress effects. It nourishes the forces of nature, allowing a person to deal with severe stress, and it reinforces emotional strength according to need. Ashwagandha can be called an "adaptogen" in modem language. Further, it enhances the forces of nature, allowing a person to deal with stress resulting from a physical or mental fatigue caused by study or work (see Archana, R., and Namasivayarn, A., 1999, J Ethnopharmacol 64, 91-93; Singh, B., Chandan, B. K., Sharma, N., Singh, S., Khajuria, A., and Gupta, D. K., 2005, Phytomedicine 12, 468-481; Bhattacharya, S. K., and Muruganandam, A. V., 2003, Pharmacol Biochem Behav 75, 547-555, the disclosure of which is incorporated herein by reference).

### (Anti-inflammatory effects)

Ashwagandha has anti-inflammatory effects and alleviates joint pain. Also, the warm sensation or penetrability of Ashwagandha improves the blood circulation in the body. Thus, Ashwagandha is often used for treatment of fever onset, arthritis, tumor, and various infectious diseases. Ashwagandha promotes maintenance of physical strength, energy, and endurance (see Anbalagan, K., and Sadique, J., 1981, Indian J Exp Biol 19, 245-249; al-Hindawi, M. K., al-Khafaji, S. H., and Abdul-Nabi, M. H., 1992, J Ethnopharmacol 37, 13-116; Agarwal, R., Diwanay, S., Patki, P., and Patwardhan, B., 1999, J Ethnopharmcol 67, 27-35; Rasool, M., and Varalakshmi, P., 2006, Vascul Pharmacol 44, 406-410, the disclosure of which is incorporated herein by reference), and the effects thereof can be equivalent to those of ginseng.

### (Antioxidant effects)

Ashwagandha is an important antioxidant nutrient. In particular, it is verified that Ashwagandha suppresses damage caused by free radicals in brain cells. Chemical substances contained in Ashwagandha are considered to be potent antioxidants, and such substances increase three antioxidant enzymes in the body (i.e., superoxide dismutase, catalase, and glutathione peroxidase) (see Bhattacharya, S. K., Satyan, K. S., and Ghosal, S., 1997, Indian J Exp Biol 35, 236-239; Bhattacharya, A., Ghosal, S., and Bhattacharya, S. K., 2001, J Ethnopharmacol 74, 1-6; Russo, A., Izzo, A. A., Cardile, V, Borrelli, F., and Vanella, A., 2001, Phytomedicine 8, 125-132, the disclosure of which is incorporated herein by reference).

### (Antibacterial activity)

Alcohol and water extracts of Ashwagandha are approved to have a wide range of antibacterial activities (see Owais, M., Sharad, K. S., Shehbaz, A., and Saleemuddin, M., 2005, Phytomedicine 12, 229-235; Arora, S., Dhillon, S., Rani, G., and Nagpal, A., 2004, Fitoterapia 75, 385-388, the disclosure of which is incorporated herein by reference).

### (Diabetes prevention)

Ashwagandha is capable of lowering the blood glucose level and cholesterol level, thereby maintaining good cardiovascular fitness (see Hemalatha, S., Wahi, A. K., Sing, P. N., and Chansouria, J. P., 2004, J Ethnopharmacol 93, 261-264; Parihar, M. S., Chaudhary, M., Shetty, R., and Hemnani, T., 2004, J Clin Neurosci 11, 397-402, the disclosure of which is incorporated herein by reference).

### (Arthritis and menstrual disorders)

Ashwagandha is considered to be effective for treatment of arthritis and menstrual disorder (see Begum, V. H., and Sadique, J., 1988, Indian J Exp Biol 26, 877-882, the disclosure of which is incorporated herein by reference).

### (Aphrodisiac effects)

Ashwagandha functions as an aphrodisiac agent, particularly for male reproductive functions, and it arouses healthy sexual impulses. Further, Ashwagandha restores reproductive functions while promoting mental stability and extends overall vigor (see Ilayperuma, I., Ratnasooriya, W. D., and Weerasooriya, T. R., 2002, Asian J Androl 4, 295-298, the disclosure of which is incorporated herein by reference).

### (Stimulation of nerves)

Ashwagandha is a *Rasayana* that reinforces the mind and has effects of reinforcing actions. It improves memory and problem-solving skills, and it enhances and reinforces well-balanced functionality throughout the brain. Further, Ashwagandha nourishes the nervous system, strengthens the nerves, and maintains neural functions.

Indian people have prescribed Ashwagandha as a therapeutic agent for brain disorders of elderly people with cognitive impairment for a long period of time (see Jain, S., Shukla, S. D., Sharma, K., and Bhatnagar, M., 2001, Phytother Res 15, 544-548; Shukla, S. D., Jain, S., Sharma, K., and Bhatnagar, M., 2000, Indian J Exp Biol 38, 1007-1013, the disclosure of which is incorporated herein by reference).

### (Therapeutic agent for old-age diseases)

Ashwagandha is used for health improvement and disease prevention for elderly people in particular (see Mishra, L. C., Singh, B. B., and Dagenais, S., 2000, Altern Med Rev 5, 334-346, the disclosure of which is incorporated herein by reference).

### (Chemotherapy)

Ashwagandha is considered to be effective for treatment of patients receiving radiation therapy or chemotherapy (see Prakash, J., Gupta, S. K., Kochupillai, V., Singh, N., Gupta, Y. K., and Joshi, S., 2001, Phytother Res 15, 240-244; Prakash, J., Gupta, S. K., and Dinda, A. K., 2002, Nutr Cancer 42, 91-97; Park, E. J., and Pezzuto, J. M., 2002, Cancer Metastasis Rev 21, 231-255, the disclosure of which is incorporated herein by reference).

At first, Ashwagandha roots were generally used in Ayurvedic medicine in India. The present inventors have investigated whether or not extracts of Ashwagandha leaves had efficacy, because leaves could be collected more easily and in larger amounts than roots. The present inventors have already demonstrated that extracts of Ashwagandha leaves obtained with the use of an organic solvent have antimutagenic effects (see Kaur, K., Rani, G., Widodo, N., Nagpal, A., Taira, K., Kaul, S. C., and Wadhwa, R., 2004, Food Chem Toxicol 42, 2015-2020; Rani, G., Kaur, K., Wadhwa, R., Kaul, S. C., and Nagpal, A., 2004, Food and Chemical Toxicology in press, the disclosure of which is incorporated herein by reference). In addition, leaf extracts obtained with the use of an organic solvent were found to specifically kill cancer cells (see Widodo, N., Kaur, K., Shreshtha, B., Takagi, Y., Ishii, H., Kaul, S. C., and Wadhwa, R., 2006, Clinc. Cancer. Res; WO 2005/082392, the disclosure of which is incorporated herein by reference).

In the past, extracts of Ashwagandha leaves that were known to have pharmacological effects were limited to those obtained with the use of an organic solvent such as methanol or hexane, Extracts obtained with the use of an organic solvent, however, are problematic for treatment or prevention of diseases due to the influence of residual organic solvents or other factors. In addition, the step of extraction involving the use of an organic solvent is complicated, and the influences of organic solvents on workers and equipment cause problems.

Accordingly, it is an object of the present invention to overcome the above-described drawbacks of conventional techniques. More specifically, it is an object of the present invention to provide a composition comprising, as an active ingredient, an extract of pharmacologically effective Ashwagandha leaves obtained without the use of an organic solvent and a method for producing the same.

### Disclosure of the Invention

The present inventors have conducted concentrated studies in order to attain the above objects. As a result, they succeeded in obtaining a water extract of Ashwagandha leaves having sufficient pharmacological effects by adding Ashwagandha leaves to water and treating the same at adequate temperature for an adequate period of time. The present invention has been completed based on such finding.

Thus, the present invention provides a composition for inhibiting or preventing aging or treating or preventing tumor or cancer, which comprises, as an active ingredient, a water extract of Ashwagandha leaves.

Another aspect of the present invention provides a composition for promoting the proliferation of normal cells or prolonging the life of the same, inducing the proteasomal activity of normal cells, protecting normal cells from stress caused by oxidation or UV irradiation, killing cancer cells or tumor cells or inhibiting the proliferation of the same, or increasing the sensitivity of cancer cells to an anticancer agent, which comprises, as an active ingredient, a water extract of Ashwagandha leaves.

Preferably, the composition according to the present invention is a food, nutritional supplement, pharmaceutical product, quasi-drug, or cosmetic product.

Preferably, in the composition according to the present invention, the water extract of Ashwagandha leaves is obtained by: a step of adding Ashwagandha leaves to water and treating the same at 20°C to 70°C for 6 to 100 hours; or a step of adding Ashwagandha leaves to water, heating the mixture to 70°C to 100°C, and then letting the resultant cool to 1°C to 30°C.

Another aspect of the present invention provides a method for producing the composition according to the present invention which comprises, a step of adding Ashwagandha leaves to water and treating the same at 20°C to 70°C for 6 to 100 hours; or a step of adding Ashwagandha leaves to water, heating the mixture to 70°C to 100°C, and then letting the resultant cool to 1°C to 30°C.

### Effects of the Invention

The present invention provides a water extract of Ashwagandha leaves having effects of promoting the proliferation of normal cells and prolonging the life of the same, inducing proteasomal activity of normal cells, protecting normal cells from stress caused by oxidation and UV irradiation, killing cancer cells and tumor cells and inhibiting the proliferation of the same, and increasing the sensitivity of cancer cells to anticancer agents. Further, the composition of the present invention enables inhibition and prevention of aging and treatment and prevention of tumor and cancer. Furthermore, the production method of the present invention can provide a composition comprising, as an active ingredient, a water extract of Ashwagandha leaves, which is used for pharmaceutical, health, quasi-drug, or cosmetic applications aimed at anti-aging or suppression of cancer or tumor formation, in large quantities and in a simple and rapid manner, with the use of Ashwagandha leaves alone or in combination with other substances.

### Brief Description of the Drawings

Fig. 1 shows the results of assaying specific anti-cancer cell activity of WEX-1.
Fig. 2 shows the results of assaying specific anti-cancer cell activity of WEX-2.
Fig. 3 shows the results of treatment of human normal cells and cancer cells with WEX-3.
Fig. 4 shows the results of assaying specific cancer-cell-killing activity of WEX-1 or WEX-2.
Fig. 5 shows the results of tumor suppression in a nude mouse *in vivo* caused by WEX-1.
Fig. 6 shows the results of assaying higher survival activity and long-term proliferation activity of human normal cells realized by WEX-1.
Fig. 7 shows the results of assaying proteasomal activity of WEX-1 in human normal cells.
Fig. 8 shows the results of assaying effects of WEX-1 for protecting human normal cells from damage caused by oxidation.
Fig. 9 shows the results of assaying the p53 level of UV-damaged cells treated with WEX-1.
Fig. 10 shows the results of assaying the activity of WEX-1 for inducing mortalin derived from normal cells or tumor cells.
Fig. 11 shows the results of HPLC and SDS-PAGE assays of WEX-1.
Fig. 12 shows the results of HPLC assay of WEX-1 under conditions in which Withanone is detected showing that WEX-1 does not contain withanone

### Embodiments for Carrying out the Invention

Hereafter, the embodiments of the present invention are described in detail.

The composition of the present invention comprises, as an active ingredient, a water extract of Ashwagandha leaves, and it has the effects of promoting the proliferation of normal cells and prolonging the life of the same, inducing the proteasomal activity of normal cells, protecting normal cells from stress caused by oxidation and UV irradiation, killing cancer cells and tumor cells and inhibiting the proliferation of the same, and increasing the sensitivity of cancer cells to an anticancer agent. Such composition is used for inhibiting or preventing aging or treating or preventing tumor or cancer. The composition of the present invention can be in the form of, for example, a food, nutritional supplement, pharmaceutical product, quasi-drug, or cosmetic product.

The term "Ashwagandha" used herein refers to *Withania somnifera* (scientific name). Accordingly, the term "Ashwagandha leaves" used herein refers to leaves of *Withani somnifera*, and the term "water extract of Ashwagandha leaves" refers to an extract obtained via treatment of Ashwagandha leaves with water.

Ashwagandha leaves may be in the state of collected fresh leaves, dried leaves, with dried leaves being preferable. Ashwagandha as a raw material is not limited to Ashwagandha growing in nature, and it may be cultured *in vitro.* Since components of Ashwagandha leaves are considered to vary to some extent depending on the locality, the age, and other conditions of Ashwagandha trees, it is preferable that 1- to 4-year-old plants cultivated from seeds in India be used in order to obtain the water extract of Ashwagandha leaves of the present invention.

The water extract of Ashwagandha leaves can be obtained by either, for example, a step of adding Ashwagandha leaves to water and treating the same at 20°C to 70°C for 6 to 100 hours or a step of adding Ashwagandha leaves to water, heating the mixture to 70°C to 100°C, and then letting the resultant cool to 1°C to 30°C.

The term "water" used herein is not particularly limited, provided that it refers to generally used water. Examples thereof include tap water and pharmaceutical water. The term "a step of adding Ashwagandha leaves to water and treating the same at 20°C to 70°C for 6 to 100 hours" used herein refers to a step of adding Ashwagandha leaves to water to obtain a water extract of Ashwagandha leaves. Such step is carried out at 20°C to 70°C, preferably 30°C to 60°C, and more preferably 40°C to 50°C for 6 to 100 hours, preferably 8 to 48 hours, and more preferably 12 to 24 hours. The step may be carried out without particular limitation within the temperature and duration ranges described above. More specifically, the above step may be carried out by, for example, mixing 10 g of dry powder of Ashwagandha leaves (native to India, purchased from iGENE) with 100 ml of water to prepare a turbid solution containing Ashwagandha leaves at a concentration of 10% herein, and slowly agitating the turbid solution in an incubator at 45°C overnight as described in the examples.

The term "a step of adding Ashwagandha leaves to water, heating the mixture to 70°C to 100°C, and then letting the resultant cool to 1°C to 30°C" used herein refers to a step of adding Ashwagandha leaves to water in order to obtain a water extract of Ashwagandha leaves as described above. Such step is carried out via heating at 70°C to 100°C, preferably 70°C to 90°C, and more preferably 80°C to 90°C and cooling at 1°C to 30°C, preferably 15°C to 30°C, more preferably 20°C to 30°C, and most preferably 20°C to 25°C. The duration of heating or cooling is not particularly limited. The above step may be carried out once or repeated two or more times, provided that a water extract of Ashwagandha leaves can be obtained. During rapid heating (e.g., heating to 80°C to 90°C within 1 to 10 minutes), for example, the step is preferably repeated two or more times, The above step may be carried out without particular limitation within the heating and cooling temperature ranges described above. A specific example of such step is a step that is carried out by repeating, twice, a cycle of mixing 10 g of dry powder of Ashwagandha leaves (native to India, purchased from iGENE) with 100 ml of water to prepare a turbid solution containing Ashwagandha leaves at a concentration of 10% therein, heating the turbid solution to about 80°C to 90°C in a microwave, and cooling the resultant to room temperature.

In either the step of adding Ashwagandha leaves to water and treating the same at 20°C to 70°C for 6 to 100 hours or the step of adding Ashwagandha leaves to water, heating the mixture to 70°C to 100°C, and then letting the resultant cool to 1°C to 30°C, a process of stirring can be additionally performed according to need. In addition to agitation, mechanical stirring using a magnetic stirrer or vortex mixer may be employed as a stirring process. Further, bacteria elimination or sterilization, such as aseptic filtration or UV sterilization, may be additionally carried out before or after the above step. When bacteria elimination or sterilization is additionally carried out before the above step, it is preferable that such step be carried out aseptically. As long as a water extract of Ashwagandha leaves can be obtained, the method of production according to the present invention can perform a variety of operations or treatments according to need, in addition to stirring, bacteria elimination, or sterilization. The water extract of Ashwagandha leaves may be adequately concentrated and purified via, for example, drying, such as hot-air drying or lyophilization, or column chromatography.

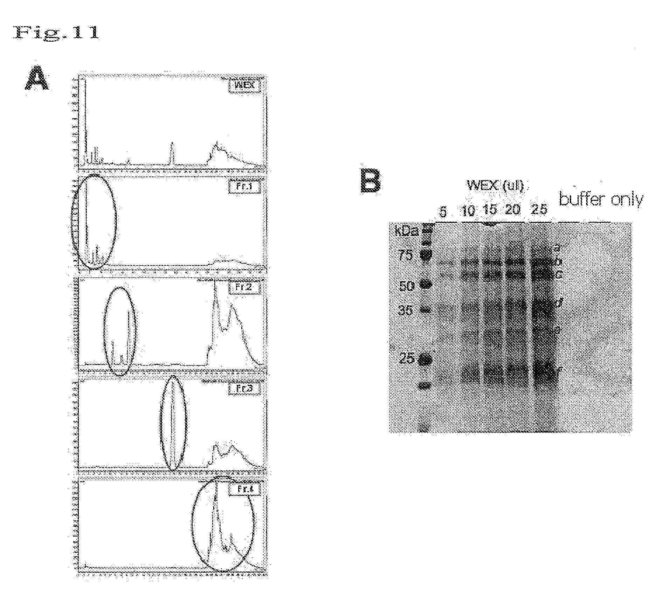

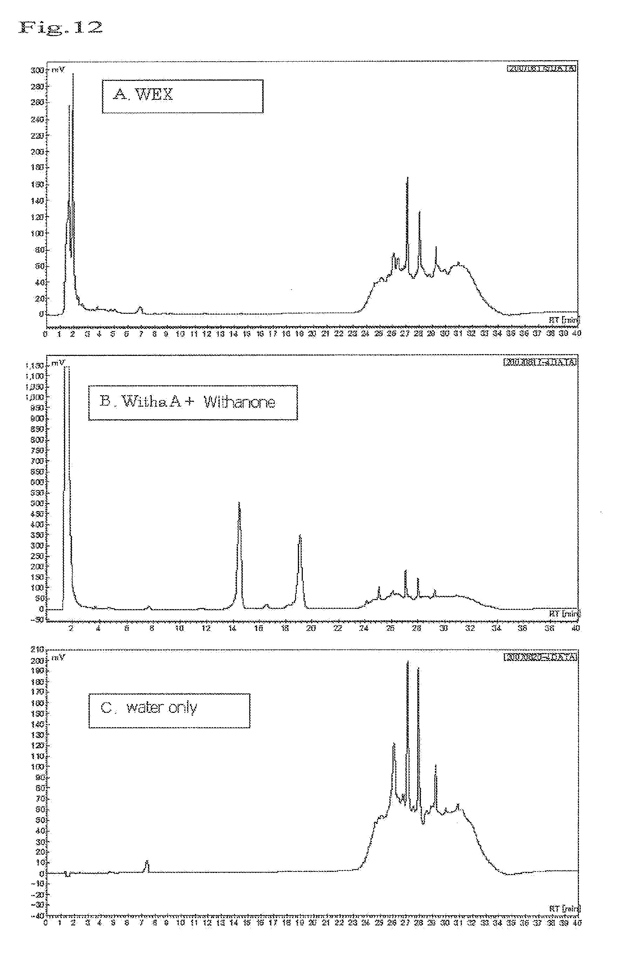

The water extract of Ashwagandha leaves comprises the compound shown in Fig. 11A or at least 6 proteins shown in Fig. 11B. As shown in Fig. 12, further, the water extract of Ashwagandha leaves differs from Withanone obtained from Ashwagandha leaves via extraction with an organic solvent. Accordingly, it is highly likely that the effects of promoting the proliferation of normal cells and prolonging the life of the same, inducing the proteasomal activity of normal cells, protecting normal cells from stress caused by oxidation and UV irradiation, killing cancer cells and tumor cells and inhibiting the proliferation of the same, and increasing the sensitivity of cancer cells to an anticancer agent of the water extract of Ashwagandha leaves are derived from one or more types of aqueous compounds (proteins) contained in the water extract of Ashwagandha leaves, which are different from Withanone.

When the composition of the present invention is a pharmaceutical composition, the route of administration thereof is not particularly limited. The route of administration can be, for example, oral, transnasal, parenteral, transpulmonary, percutaneous, or transmucosal. The pharmaceutical composition of the present invention can be in a variety of dosage forms. Examples of dosage forms for oral administration include, but are not limited to, tablets, capsules, powders, granules, pills, liquid preparations, emulsions, suspensions, solutions, spirits, syrups, extracts, and elixirs. Also, a variety of pharmacologically acceptable carriers can be added to the preparations. Examples thereof include, but are not limited to, excipients, binders, disintegrators, lubricants, flavors, colorants, sweeteners, flavoring substances, solubilizers, suspending agents, emulsifiers, coating agents, vitamin C, and antioxidants.

The dose of the pharmaceutical composition of the present invention is generally 1 mg to 1,000 mg and preferably 100 mg to 500 mg in terms of the extract of Ashwagandha leaves per day per adult. It can be individually varied depending on the age, the body weight, and symptoms of a patient, the route of administration, the duration of administration, the course of treatment, or other conditions. The dose per day can be administered in several separate instances. The pharmaceutical composition can be administered in combination with other anti-tumor agents or another treatment technique.

The composition of the present invention can be in the form of a food or nutritional supplement. For example, the extracts of Ashwagandha leaves may be incorporated into raw materials so as to prepare noodles, bread, candies, jellies, cookies, soup, healthy drinks, or an alcoholic beverage such as *shochu* (a distilled spirit). Such food or nutritional supplement may further comprise inorganic components such as iron or calcium, various vitamins, dietary fibers such as oligosaccharide or chitosan, proteins such as soybean extracts, lipids such as lecithin, and sugars such as sucrose or lactose, in addition to the extracts of Ashwagandha leaves.

The composition of the present invention can be prepared in the form of a quasi-drug or cosmetic product by incorporating the extracts of Ashwagandha leaves into raw materials. When the composition of the present invention is prepared in the form of a quasi-drug or cosmetic product, other ingredients that are generally used for quasi-drugs or cosmetic products can be adequately added in addition to the extracts of Ashwagandha leaves, provided that the effect of the present invention is not lost. For example, oils, wetting agents, ultraviolet absorbers, antioxidants, surfactants, antiseptics, moisturizing agents, aroma chemicals, water, alcohol, or thickeners may be added according to need.

The composition of the present invention has the effects of promoting the proliferation of normal cells and prolonging the life of the same, inducing the proteasomal activity of normal cells, protecting normal cells from stress caused by oxidation and UV irradiation, killing cancer cells and tumor cells and inhibiting the proliferation of the same, and increasing the sensitivity of cancer cells to an anticancer agent, and the composition enables suppression and prevention of aging and treatment and prevention of tumor and cancer. Whether or not the composition of the present invention has the effects of promoting the proliferation of normal cells and prolonging the life of the same, inducing the proteasomal activity of normal cells, protecting normal cells from stress caused by oxidation and UV irradiation, killing cancer cells and tumor cells and inhibiting the proliferation of the same, and increasing the sensitivity of cancer cells to an anticancer agent or whether or not the composition enables suppression and prevention of aging and treatment and prevention of tumor and cancer can be determined by any method described in the examples below, although the methods are not limited thereto.

The present invention is described in greater detail with reference to the following examples, although the present invention is not limited thereto. Those skilled in the art can implement various forms of variations or modifications, and such variations or modifications are within the scope of the present invention.

### Example 1

### 1. Preparation of water extract (WEX) from Ashwagandha leaves

Dry powder of Ashwagandha leaves (10 g, native to India, purchased from iGENE) was mixed with 100 ml of water to prepare a 10% turbid solution. Subsequently, one of the following treatments was carried out.
(1) The turbid solution was slowly agitated in an incubator at 45 °C overnight.
(2) The turbid solution was heated to about 80°C to 90°C in a microwave oven and was then cooled to room temperature. The turbid solution was heated again to about 80°C to 90°C in a microwave oven and was then cooled to room temperature.
(3) The turbid solution was treated in an autoclave at 120°C for 20 minutes and was then cooled to room temperature. The turbid solution was treated in an autoclave at 120°C for 20 minutes again and was then cooled to room temperature.

The turbid solution subjected to any of treatments (1) to (3) was centrifuged at 10,000 rpm for 20 minutes, and the supernatant was faltered through a 0.45-µm filter. The aqueous filtrate was fractionated to 1 - to 2-ml fractions and cryopreserved at -20°C before the experiment. Hereafter, the turbid solution subjected to treatment (1) is referred to as "WEX-1," the solution subjected to treatment (2) is referred to as "WEX-2," and the solution subjected to treatment (3) is referred to as "WEX-3."

### Example 2

### 2. Specific anti-cancer cell activity of WEX-1 and WEX-2

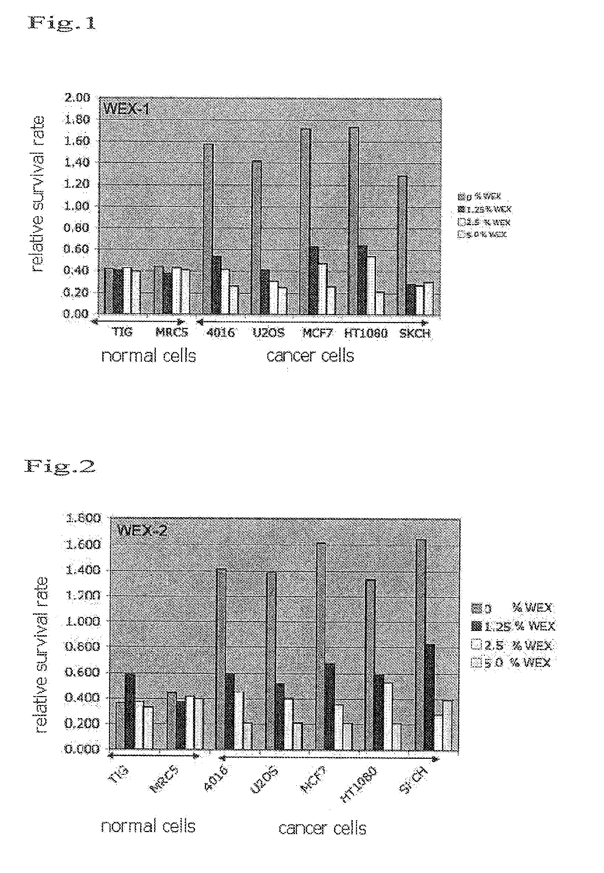

In Dulbecco's Modified Eagle's Minimum Medium (hereafter abbreviated as "DMEM," GIBCO BRL, U.S.A.) containing 10% fetal bovine serum (hereafter abbreviated as "FBS"), normal human diploid fibroblasts (TIG-1 and MRC5), various cancer cells (osteosarcoma-derived U20S, breast carcmoma-derived MCF7, fibrosarcoma-derived HT1080, colon cancer-derived HCT116 (40-16), and gallbladder cancer-derived SKCH) were cultured with the use of a 96-well culture plate in a humidified incubator (37°C, 5% CO₂). Cells (3 × 10⁵ cells to 5 × 10⁵ cells) were treated with WEX-1 or WEX-2. The viable cell counts were monitored with the use of the WST cell proliferation assay kit (Roche) (Fig. 1 and Fig. 2). As a result, WEX-1 and WEX-2 were found to kill human cancer cells but not to affect normal human cells.

### Example 3

### 3. Anti-cancer cell activity of WEX-3

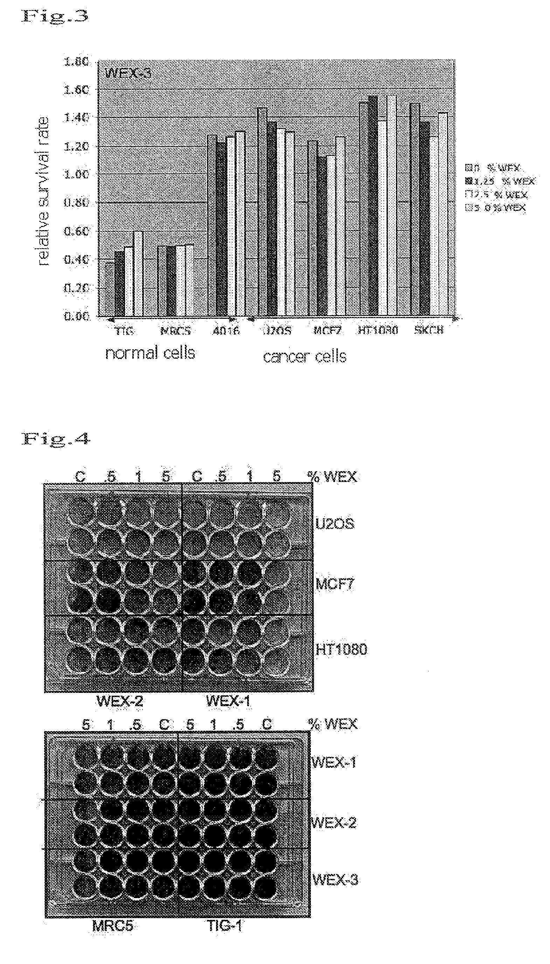

In the same manner as in Example 2, human normal cells and cancer cells were treated with WEX-3 (Fig. 3). As a result, WEX-3 was found to have no influence on cancer cells or normal cells. WEX-3 was found to lack the activity observed in WEX-1 and WEX-2. This demonstrates that treatment with WEX-1 under high pressure at a high temperature would decrease the effects of killing cancer cells.

### Example 4

### 4. Cancer-cell-killing activity of WEX-1 and WEX-2

Cells were cultured in the same manner as in Example 2. After the cells were treated with WEX, the cells were immobilized with ice-chilled methanol (stored at -20°C) for 2 to 3 minutes, washed with PBS, and then soaked in Crystal Violet (a solution of 0.5% Crystal Violet in 25% methanol) for about 1 to 2 hours. The cells were excessively rinsed with water, dried, and photographed (Fig. 4). As a result, proliferation of cancer cells (U2OS, MCF7, and HT1080) was terminated via treatment with WEX-1 and WEX-2. Normal cells (TIG-1 and MRC5) did not show any difference in terms of cell proliferation. Thus, WEX-1 and WEX-2 were found to have activity of killing cancer cells and could be used to prepare safe aqueous solutions for normal cells.

### Example 5

### 5. In vivo anti-tumor assay using WEX-1

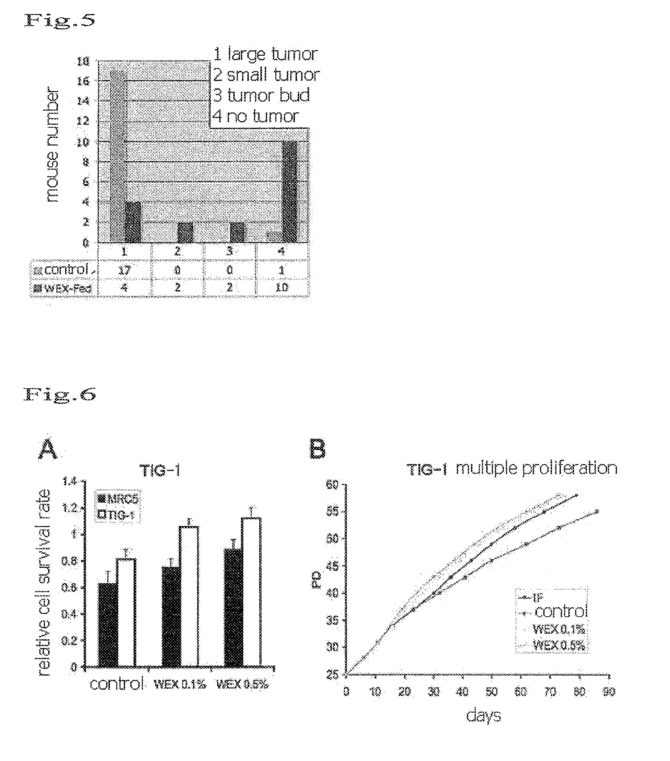

In order to perform *in vivo* tumor assay, human fibrosarcoma (HT1080)-derived cells (1×10⁶ cells in 0.5 ml growth medium) were injected hypodermically through the flanks of Balb/c nude mice (2 sites per mouse). Tumorigenesis of the control mice, mice into which WEX had been injected (local injection into tumor sites), and mice to which WEX had been fed (cases in which WEX was orally administered every other day with the use of 2% sterile carboxymethyl cellulose (CMC) as a carrier and a movable Teflon® injector for injection into the alimentary tracts of mice) was monitored (Fig. 5). As a result, local injection of WEX-1 into the tumor site and WEX-1 feeding were found to suppress tumors via *in vivo* assay of nude mice.

### Example 6

### 6. In vitro longevity studies

Normal human fibroblasts were cultured in the presence of WEX-1 in a short-term viability assay (A) or a long-term serial passage assay (B) (Fig. 6). The cells cultured in the presence of WEX-1 exhibited higher viability (A), and exhibited increase in long-term proliferation capacity (B).

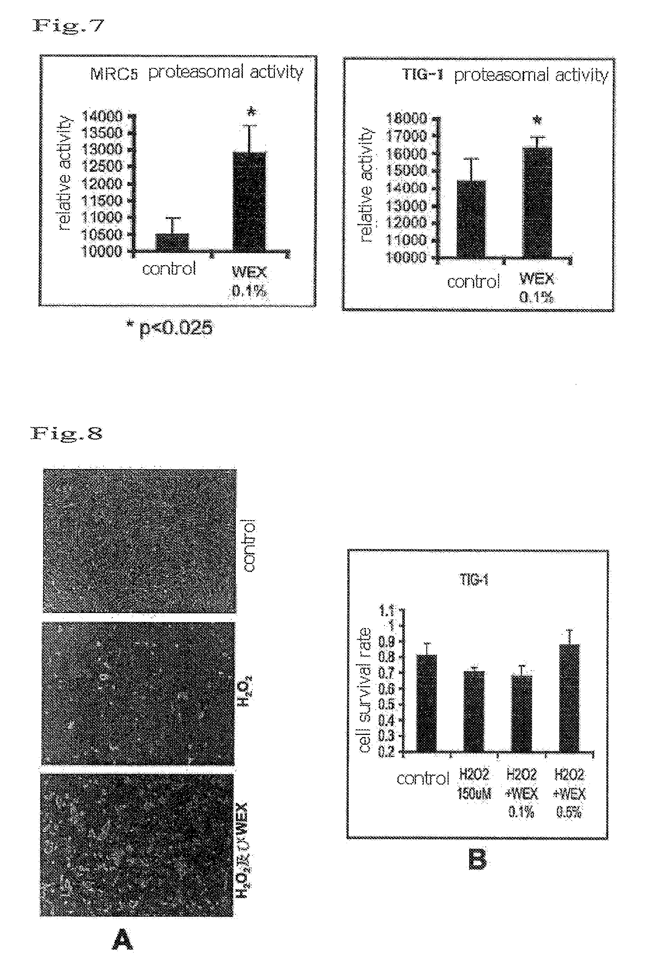

Normal human fibroblasts (TIG-1 and MRC5) were cultured in the presence of WEX-1, and proteasomal activity was assayed (Fig. 7). Treatment with WEX-1 was found to induce proteasomal activity in both types of normal cells used.

### Example 7

### 7. Protection of normal cells from damage caused by oxidation

Normal fibroblasts (TIG-1) were exposed to stress caused by oxidation via treatment with hydrogen peroxide, and restoration was inspected in the presence or absence of WEX-1 (Fig. 8). As a result, normal cells were found to protect themselves or to recover from damage caused by oxidation in the presence of WEX-1.

### Example 8

### 8. Protection of normal cells from UV damage

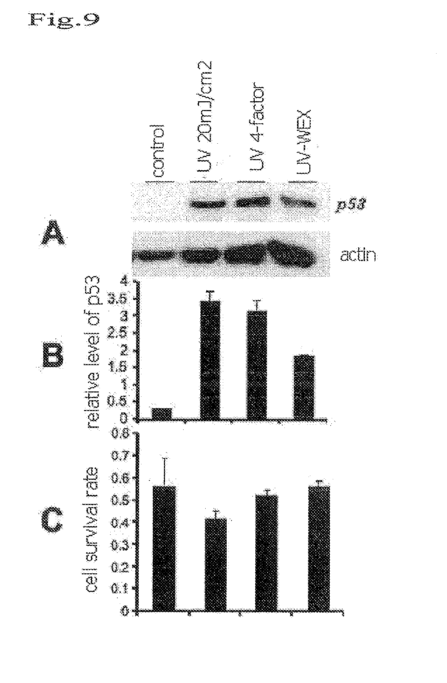

Normal fibroblasts (TIG-1) were exposed to UV stress (20 mJ/cm²). The p53 protein level, which is a molecular marker of UV damage, was inspected via Western blotting, and the viable cell count was inspected via WST assay (Fig. 9). As a result, the cells treated with WEX-1 were found to have lowered p53 protein levels. The i-Factor (Withanone) was used as a positive control (A-B). The viable cell count also increased in the presence of WEX-1.

### Example 9

### 9. Induction of mortalin stress chaperone only in normal cells

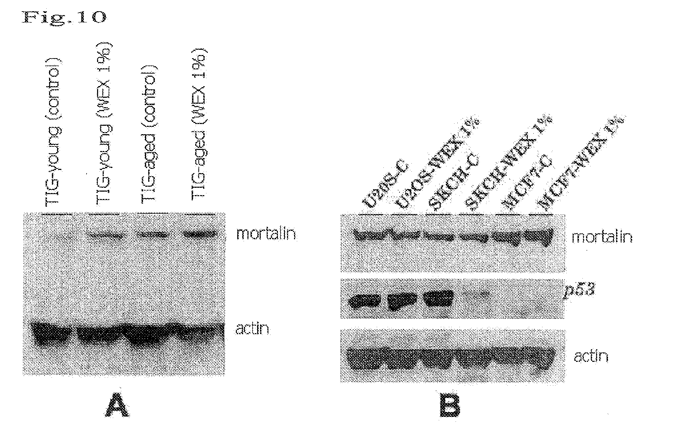

Normal fibroblasts (TIG-1; young cells and aged cells) and tumor-derived cells (U2OS, SKCH, and MCF7) were treated with WEX for 48 to 72 hours, and the stress protein and p53 protein levels were inspected via Western blotting (Fig. 10). As a result, WEX-1 was found to induce mortalin only in normal cells (A). Interestingly, the amounts of the mutant p53 proteins were dramatically decreased via treatment with WEX in SKCH cells (B).

### Example 10

### 10. Fractionation and analysis of WEX-1

As described below, WEX-1 was analyzed via HPLC and via SDS-PAGE. WEX was fractionated via reversed-phase HPLC using the C18 column (TSKgel ODS-100Z, Tosoh Corporation). Reversed-phase HPLC was carried out at a flow rate of 1 ml/min and a column temperature of 40°C, and gradient extraction was carried out with the use of water (solution A) and ethanol (solution B). The 35-minute gradient program is as described below: a constant level of solution A at 100% for 5 minutes; gradient to 0.75% of solution B for 15 minutes, gradient from 0.75% to 50% of solution B for 5 minutes, a constant level of solution B at 50% for 5 minutes, gradient from 50% to 0% of solution B for 2 minutes, and a constant level of solution A at 100% for 5 minutes in the end. Detection was carried out at 220 nm (Fig. 11). As shown in Fig. 11A, WEX was consequently fractionated to 4 constructs (Fractions 1 to 4). WEX-1 was separated on SDS PAGE gel and subjected to staining with Coomassie brilliant blue. As shown in Fig. 11B, WEX contained 6 proteins, which were labeled "a" to "f." This indicates that WEX contains large numbers of water-soluble proteins, which are not able to be present via extraction of Withanone with the use of as organic solvent.

### Example 11

### 11. Composition analysis of extract

HPLC analysis was carried out under conditions in which Withanone could be detected as described below (Fig. 12).
Column: Symmetry® C-18 (5 µm 150 (1) × 4.6 (d))
Column oven: 40°C
Solution A: 1% methanol
Solution B : methanol: ethanol: i-propanol = 52.25 45.30: 2.45
Gradient program:
Initial: A65% (B35%)
→ 20 min: A59% (B41%)
→ 23 min: A0% (B100%)
→ 28 min: A0% (B100%)
→ 31 min: A65% (B35%)
→ 36 min: A65% (B35%)
Flow rate: 1 ml/min
Detector wavelength: 220 nm

As shown in Fig. 12, Withanone could not be detected in WEX. The results indicate that WEX is a water extract, but Withanone cannot be obtained via extraction with water because of the chemical properties thereof (i.e., Withanone is low in solubility and hardly soluble in water).

## Claims

1. A composition for inhibiting or preventing aging or treating or preventing tumor or cancer, which comprises, as an active ingredient, a water extract of Ashwagandha leaves.

2. A composition for promoting the proliferation of normal cells or prolonging the life of the same, inducing the proteasomal activity of normal cells, protecting normal cells from stress caused by oxidation or UV irradiation, killing cancer cells or tumor cells or inhibiting the proliferation of the same, or increasing the sensitivity of cancer cells to an anticancer agent, which comprises, as an active ingredient, a water extract of Ashwagandha leaves.

3. The composition according to claim 1 or 2, which is a food, nutritional supplement pharmaceutical product, quasi-drug, or cosmetic product.

4. The composition according to any one of claims 1 to 3, wherein the water extract of Ashwagandha leaves is obtained by:
a step of adding Ashwagandha leaves to water and treating the same at 20°C to 70°C for 6 to 100 hours; or
a step of adding Ashwagandha leaves to water, heating the mixture to 70°C to 100°C, and then letting the resultant cool to 1°C to 30°C.

5. A method for producing the composition according to any one of claims 1 to 4 which comprises:
a step of adding Ashwagandha leaves to water and treating the same at 20°C to 70°C for 6 to 100 hours; or
a step of adding Ashwagandha leaves to water, heating the mixture to 70°C to 100°C, and then letting the resultant cool to 1°C to 30°C.
